# EUROPEAN PATENT SPECIFICATION

(11) **EP 4 472 723 B1**
(45) Date of publication and mention of the grant of the patent: **28.10.2026**
(21) Application number: 23708954.5
(22) Date of filing: 01.02.2023
(51) Int. Cl.: A61N 1/05, A61N 1/36

(54) **NEUROSTIMULATION ADAPTED TO INTRINSIC FREQUENCY DRIFT**
AN INTRINSISCHE FREQUENZDRIFT ANGEPASSTE NEUROSTIMULATION
NEUROSTIMULATION ADAPTÉE À UNE DÉRIVE DE FRÉQUENCE INTRINSÈQUE

(30) Priority: 04.02.2022 US 202263306706 P
(43) Date of publication of application: 11.12.2024
(73) Proprietor: Boston Scientific Neuromodulation Corporation, Valencia, CA 91355 (US)
(72) Inventor: BOKIL, Hemant, Cambridge, MA 02138 (US); CARCIERI, Stephen, Los Angeles, CA 90026 (US); YOO, Peter J., Burbank, CA 91504 (US)
(74) Representative: Vossius & Partner Patentanwälte Rechtsanwälte mbB
(86) International application number: PCT/US2023/012104
(87) International publication number: WO 2023/150157

(56) References cited:
- US-A1- 2016 287 879
- US-B2- 10 820 819
- US-B2- 9 168 374

## Description

### TECHNICAL FIELD

This document relates generally to medical devices, and more particularly to system for adapting neurostimulation to frequency drift of an intrinsic oscillatory neural activity in a patient.

### BACKGROUND

Neurostimulation, also referred to as neuromodulation, has been proposed as a therapy for a number of conditions. Examples of neurostimulation include Spinal Cord Stimulation (SCS), Deep Brain Stimulation (DBS), Peripheral Nerve Stimulation (PNS), and Functional Electrical Stimulation (FES). Implantable neurostimulation systems have been applied to deliver such a therapy. An implantable neurostimulation system may include an implantable neurostimulator, also referred to as an implantable pulse generator (IPG), and one or more implantable leads each including one or more electrodes. The implantable neurostimulator delivers neurostimulation energy through one or more electrodes placed on or near a target site in the nervous system. An external programming device is used to program the implantable neurostimulator with stimulation parameters controlling the delivery of the neurostimulation energy.

The neurostimulation energy may be delivered in the form of electrical neurostimulation pulses. The delivery is controlled using stimulation parameters that specify spatial (where to stimulate), temporal (when to stimulate), and informational (patterns of pulses directing the nervous system to respond as desired) aspects of neurostimulation pulses. Some implantable neurostimulators have enhanced capabilities of providing multiple therapy modalities at various neural targets. For example, for DBS, electrical neurostimulation is delivered to implantable electrodes located at certain neurostimulation targets in the brain to treat various neurological or neurophysiological disorders. Effectively and efficiently searching for desirable and individualized DBS therapy remains a technological challenge in implantable neurostimulation devices.

US 9,168,374 B2 is directed to devices, systems, and techniques for delivering electrical stimulation to a patient with varying pulse frequencies within each burst of pulses. The pulse frequency variation within each burst of pulses may be between approximately 20 Hz and 40 Hz. In some examples, the pulse frequency may be increased, decreased, or polynomially varied within each burst.

### SUMMARY

Deep brain stimulation (DBS) has been used to treat or control neurodegenerative diseases such as essential tremor, Parkinson's disease, primary and secondary dystonia, among other neurological or neurophysiological disorders. DBS may be provided in a form of tonic stimulation, characterized by a consistent stream of pulses at a set frequency, pulse width, and amplitude; or in form of burst stimulation (e.g., theta burst) comprising groups of pulses at a lower amplitude and a higher frequency than tonic stimulation, followed by pulse-free periods during which charge balance occurs. For DBS, bursts arriving at specific phase(s) of an oscillation cycle of an intrinsic oscillatory neural activity in the patient may be more effective than bursts arriving at other phases of the oscillation cycle. The intrinsic oscillatory neural activity may be caused by patient underlying pathological conditions, such as essential tremor or neurodegenerative diseases such as Parkinson's disease. Although timing bursts to particular phases of an intrinsic oscillation appears a viable solution, a complication with such approach is that the frequency of that oscillation may exhibit drifts over time. In some patients, for example, the frequency of the oscillatory neural activity may change from 5 Hz to 6 Hz over a few minutes. Such frequency drift, although generally slow compared to the burst stimulation frequency, may still reduce neurostimulation therapy efficacy such as symptom control by DBS. Moreover, the frequency drift of the intrinsic oscillation may vary from patient to patient in rate and/or pattern, which makes burst stimulation calibration a complicated and time-consuming process.

The subject-matter of the present invention is defined by the features of the independent claims. Further embodiments of the present invention are defined in the dependent claims. In particular, the present document describes systems, devices, and non-claimed methods for adapting neurostimulation, such as bursts of pulses, to frequency drift of an intrinsic oscillatory neural activity in a patient. The adaptive control of neurostimulation may be achieved by adjusting one or more stimulation parameters in the presence of an indication of frequency drift, the stimulation parameters including, for example, a timing for initiating bursts at a particular phase of an oscillation cycle of the intrinsic oscillatory neural activity and a burst frequency (e.g., an inter-burst frequency, or an intra-burst frequency). The adaptive control of neurostimulation as described in this document improves the technology of device-based neurostimulation therapy. For example, it can improve the therapeutic efficacy of DBS in treating various neurological or neurophysiological disorders. The neurostimulation adapted to frequency drift of the patient intrinsic oscillatory neural activity can also improve device automaticity, therapy programming efficiency , and overall functionality of computerized implantable neuromodulation devices.

An example (e.g., "Example 1") of a system for providing neurostimulation to a neural target of a patient is provided. The system comprises an implantable stimulator and a controller. The implantable stimulation can provide burst stimulation to the neural target, such as a brain target. The burst stimulation comprises a pulse train followed by a pulse-free period. The controller can receive an indication of frequency drift of an intrinsic oscillatory neural activity in the patient, calibrate a burst stimulation parameter in response to the indication of frequency drift, and generate a control signal to the implantable stimulator to generate burst stimulation in accordance with the calibrated burst stimulation parameter. In some examples, the controller can evaluate patient responses to burst stimulation, such as levels of symptom relief in response to test stimulation bursts delivered to the neural target in accordance with respective values of a burst stimulation parameter, and determine an indication of frequency drift of an intrinsic oscillatory neural activity based on the patient response. The indication of frequency drift may be provided to a user (e.g., a clinician), or to a process such as to guide adjustment or optimization of a burst stimulation parameter.

Example 1 is a system for providing electrostimulation to a patient, comprising: an implantable stimulator configured to provide burst stimulation to a neural target of the patient, the burst stimulation comprising a pulse train followed by a pulse-free period; and a controller circuit configured to: receive an indication of frequency drift of an intrinsic oscillatory neural activity in the patient; determine or adjust a burst stimulation parameter in response to the received indication of frequency drift; and generate a control signal to the implantable stimulator to provide burst stimulation in accordance with the determined or adjusted burst stimulation parameter.

This Summary is an overview of some of the teachings of the present application and not intended to be an exclusive or exhaustive treatment of the present subject matter. Further details about the present subject matter are found in the detailed description and appended claims. Other aspects of the disclosure will be apparent to persons skilled in the art upon reading and understanding the following detailed description and viewing the drawings that form a part thereof, each of which are not to be taken in a limiting sense. The scope of the present disclosure is defined by the appended claims.

### BRIEF DESCRIPTION OF THE DRAWINGS

The drawings illustrate generally, by way of example, various embodiments discussed in the present document. The drawings are for illustrative purposes only and may not be to scale.
FIG. 1 illustrates an example of a neurostimulation system.
FIG. 2 illustrates an example of a stimulation device and a lead system that may be implemented in the neurostimulation system of FIG. 1.
FIG. 3 illustrates an example of a programming device that may be implemented in the neurostimulation system of FIG. 1.
FIGS. 4A-4B illustrate examples of an implantable pulse generator (IPG) and an implantable lead system.
FIG. 5 illustrates an example of an IPG and an implantable lead system arranged to provide brain stimulation to a patient.
FIG. 6 illustrates an example of portions of a neurostimulation system.
FIG. 7 illustrates an example of an implantable stimulator and one or more leads of an implantable neurostimulation system, such as the implantable neurostimulation system of FIG. 6.
FIG. 8 illustrates an example of an external programming device of an implantable neurostimulation system, such as the implantable neurostimulation system of FIG. 6.
FIG. 9 is a diagram illustrating an example of frequency drift of an intrinsic oscillatory neural activity in a patient, and calibration of stimulation parameters used in DBS.
FIG. 10 is a diagram illustrating an example of a prediction model representing a relationship between a quantitative measure of symptoms and stimulation parameter values.
FIG. 11 illustrates, by way of example and not limitation, a non-claimed method for providing electrostimulation to a patient in the presence of frequency drift of an intrinsic oscillatory neural activity.
FIG. 12 illustrates, by way of example and not limitation, a non-claimed method for determining or adjusting a stimulation parameter based on patient responses to test stimulation bursts in a stimulation optimization session.
FIG. 13 illustrates a block diagram of an example machine upon which any one or more of the techniques (e.g., methodologies) discussed herein may perform.

### DETAILED DESCRIPTION

In the following detailed description, reference is made to the accompanying drawings which form a part hereof, and in which is shown by way of illustration specific embodiments in which the invention may be practiced. These embodiments are described in sufficient detail to enable those skilled in the art to practice the invention, and it is to be understood that the embodiments may be combined, or that other embodiments may be utilized and that structural, logical and electrical changes may be made without departing from the scope of the present invention. References to "an", "one", or "various" embodiments in this disclosure are not necessarily to the same embodiment, and such references contemplate more than one embodiment. The following detailed description provides examples, and the scope of the present invention is defined by the appended claims and their legal equivalents.

This document discusses, among other things, a neurostimulation system for providing neurostimulation to a neural target of a patient, such as DBS bursts to a brain target. The system comprises an implantable stimulator and a controller. The implantable stimulator can provide burst stimulation comprising a pulse train followed by a pulse-free period. The controller can receive an indication of frequency drift of an intrinsic oscillatory neural activity in the patient, and calibrate a burst stimulation parameter in response to the indication of frequency drift. The frequency drift can be received as a user input, or predicted using physiological signals collected from the patient. In some examples, the frequency drift may be determined based on patient responses (e.g., symptom relief) to a series of test stimulation bursts. The burst stimulation parameter can include a burst frequency or a timing for initiating the burst stimulation at a particular phase of an oscillation cycle of the intrinsic oscillatory neural activity. The controller can generate a control signal to the implantable stimulator to generate burst stimulation in accordance with the calibrated burst stimulation parameter.

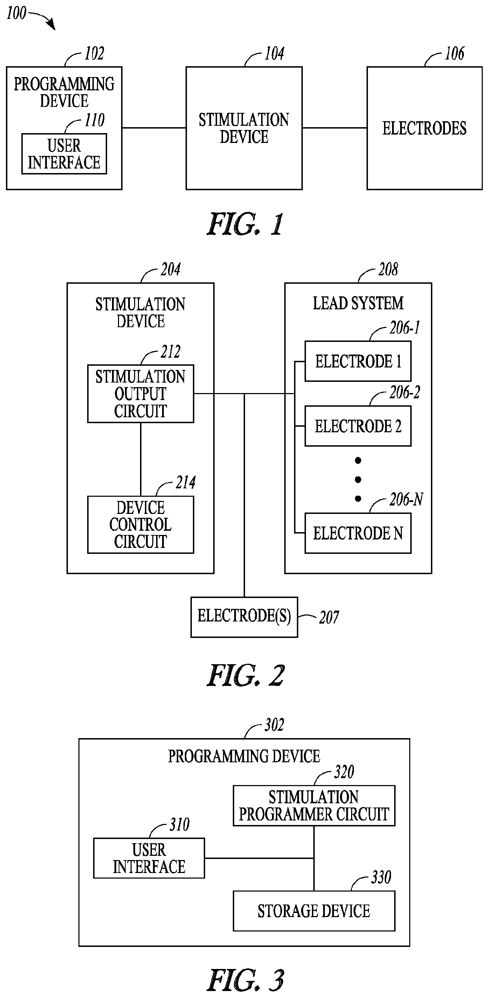

FIG. 1 illustrates, by way of example and not limitation, an embodiment of a neurostimulation system 100. In an example, the system 100 may be configured for DBS applications. Such DBS configuration includes various features that may simplify the task of the user in programming the stimulation device 104 for delivering DBS to the patient, such as the features discussed in this document.

The system 100 includes a programming device 102, a stimulation device 104, and electrodes 106. The electrodes 106 may be configured for placement on or near one or more neural targets in a patient. The stimulation device 104 may be configured to be electrically connected to the electrodes 106 and deliver neurostimulation energy, such as in the form of electrical pulses, to the one or more neural targets though the electrodes 106. In an example, the stimulation device 104 controls the delivery of neurostimulation energy according to a plurality of stimulation parameters, such as a selection of active electrodes for passing electrostimulation energy to the tissue, or stimulation pattern of the electrical pulses, among others. In various examples, at least some of the stimulation parameters are programmable by a user, such as a clinician.

The programming device 102 may be configured to be in communication with the stimulation device 104 via a wired or wireless link. The programming device 102 may provide the user with accessibility to user-programmable parameters. In the illustrated example, the programming device 102 may include a user interface 110 that allows a user to control the operation of the system 100 and monitor the performance of the system 100 as well as conditions of the patient including responses to the delivery of the neurostimulation. The user may control the operation of the system 100 by setting and/or adjusting values of the user-programmable parameters. In various examples, the user interface 110 may include a graphical user interface (GUI) that allows the user to create and/or edit graphical representations of various stimulation waveforms. The GUI may also allow the user to set and/or adjust stimulation fields each defined by a set of electrodes through which one or more neurostimulation pulses represented by a waveform are delivered to the patient. The stimulation fields may each be further defined by the current fractionalization across the set of electrodes. In various examples, neurostimulation pulses for a stimulation period (such as the duration of a therapy session) may be delivered to multiple stimulation fields.

In this document, a "user" includes a physician or other clinician or caregiver who treats the patient using the system 100; a "patient" includes a person who receives, or is intended to receive, neurostimulation via the system 100. In various examples, the patient may be allowed to adjust his or her treatment using system 100 to certain extent, such as by adjusting certain therapy parameters and entering feedback and clinical effect information.

FIG. 2 illustrates, by way of example and not limitation, an embodiment of a stimulation device 204 and a lead system 208, such as may be implemented in the neurostimulation system 100. The stimulation device 204 represents an embodiment of stimulation device 104, and includes a stimulation output circuit 212 and a device control circuit 214. The stimulation output circuit 212 may produce and deliver neurostimulation pulses. The device control circuit 214 may control the delivery of the neurostimulation pulses from stimulation output circuit 212 according to a plurality of stimulation parameters. The lead system 208 includes one or more leads each configured to be electrically connected to stimulation device 204 and a plurality of electrodes 206 (including electrode 206-1, 206-2, ..., 206-N) distributed in the one or more leads. Each of the electrodes 206 has an electrically conductive contact providing for an electrical interface between the stimulation output circuit 212 and patient tissue. In an example, the lead system 208 may include two leads each having eight electrodes.

The neurostimulation pulses are each delivered from stimulation output circuit 212 through a set of electrodes selected from electrodes 206. In various examples, the neurostimulation pulses may include one or more individually defined pulses, and the set of electrodes may be individually definable by the user for each of the individually defined pulses or each of collections of pulse intended to be delivered using the same combination of electrodes. In various examples, one or more additional electrodes 207 (referred to as reference electrodes) may be electrically connected to stimulation device 204, such as one or more electrodes each being a portion of or otherwise incorporated onto a housing of stimulation device 204. The neurostimulation may be delivered as a unipolar, bipolar, or multipolar stimulation. Monopolar stimulation uses a monopolar electrode configuration with one or more electrodes selected from the electrodes 206 and at least one electrode from electrode(s) 207. Bipolar stimulation uses a bipolar electrode configuration with two electrodes selected from the electrodes 206 and none of the electrode(s) 207. The bipolar stimulation may include balanced or unbalanced bipolar mode using a pair of electrodes on a lead, with the balancing current being applied to a reference electrode. Multipolar stimulation uses a multipolar electrode configuration with multiple (two or more) electrodes selected from electrodes 206 and none of electrode(s) 207. In various examples, the number of leads and the number of electrodes on each lead depend on, for example, the distribution of target(s) of the neurostimulation and the need for controlling the distribution of electric field at each target.

FIG. 3 illustrates, by way of example and not limitation, a programming device 302, which may be an embodiment of the programming device 102 and implemented in neurostimulation system 100. The programming device 302 may include a storage device 330, a stimulation programmer circuit 320, and a user interface 310. The stimulation programmer circuit 320 may be a part of control circuitry of the programming device 302, and is configured to support one or more functions allowing for programming of stimulation devices, such as stimulation device 104 including its various embodiments as discussed in this document. In various examples, the stimulation programmer circuit 320 may generate a plurality of stimulation parameters, collectively referred to as a stimulation configuration, that control the delivery of the neurostimulation pulses. In this document, a "stimulation configuration" may include the pattern of neurostimulation pulses including one or more stimulation fields, or at least various aspects or parameters of the pattern of neurostimulation pulses. In various examples, the stimulation configuration may specify a stimulation current (e.g., amplitude or energy of the stimulation) and an electrical current fractionalization across the plurality of electrodes. In some examples, the stimulation configuration may include a stimulation location and a stimulation current that corresponds to a best metric value. In various examples, the stimulation configuration may include a virtual electrode state that specifies a virtual electrode type, location of the virtual electrode in a coordinate space, and stimulation current associated with virtual electrode voltage field and virtual electrode location. Electrical current fractionalization across a plurality of electrodes may be determined based on the voltage field of the virtual electrode.

The storage device 330 may store information used by the stimulation programmer circuit 320, including the stimulation configuration, and information about a virtual electrode and steering of the virtual electrode on a graphical user interface. The user interface 310 represents an embodiment of user interface 110, and may be coupled to the stimulation programmer circuit 320. In various examples, the user interface 310 may allow for definition of a pattern of neurostimulation pulses for delivery during neurostimulation therapy by creating and/or adjusting one or more stimulation waveforms using a graphical method. The definition may also include definition of one or more stimulation fields each associated with one or more pulses in the pattern of neurostimulation pulses. In various examples, the user interface 310 may include a GUI that allows the user to define the pattern of neurostimulation pulses and perform other functions using graphical methods.

The circuits or subcircuits included in the neurostimulation system or devices, and their variations discussed in this document, may be implemented using a combination of hardware and software. For example, the circuit of user interface 110, device control circuit 214, stimulation programmer circuit 320, and stimulation programmer circuit 320, including their various embodiments discussed in this document, may be implemented using an application-specific circuit constructed to perform one or more particular functions or a general-purpose circuit programmed to perform such function(s). Such a general-purpose circuit includes, but is not limited to, a microprocessor or a portion thereof, a microcontroller or portions thereof, and a programmable logic circuit or a portion thereof.

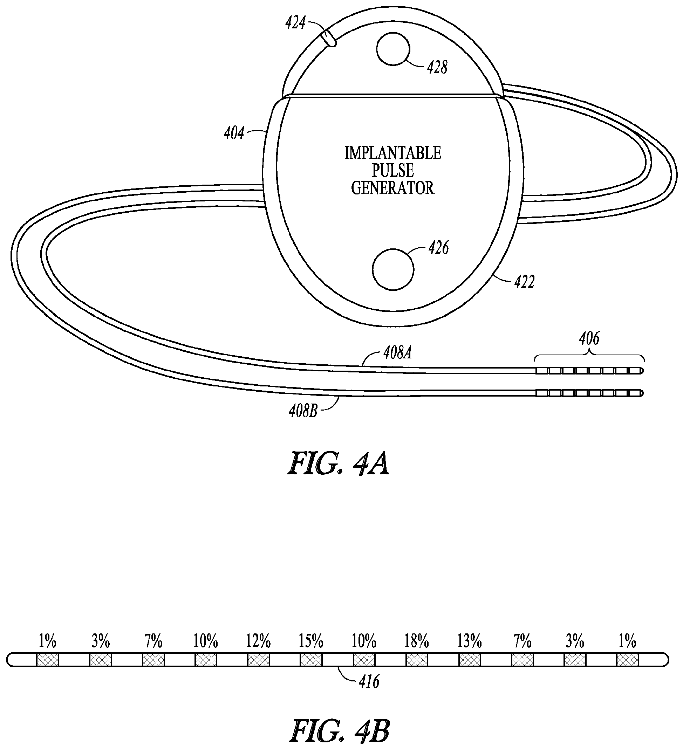

FIGS. 4A-4B illustrate, by way of example and not limitation, embodiments of an implantable pulse generator (IPG) 404 and an implantable lead system 408. The IPG 404 represents an example implementation of stimulation device 204, and may include a hermetically-sealed IPG case 422 to house the electronic circuitry of IPG 404. The IPG 404 may include an electrode 426 formed on the IPG case 433. The IPG 404 may include an IPG header 424 for coupling the proximal ends of leads 408A and 408B. The IPG header 424 may optionally include an electrode 428. Electrodes 426 and/or 428 represent embodiments of electrode(s) 207 and may each be referred to as a reference electrode. The IPG 404 may be communicatively coupled to a programming device, such as the programmer device 102 or the programming device 302, and configured to generate and deliver neurostimulation energy according to the stimulation configuration generated by the programming device 102 or 302.

The lead system 408 represents an example implementation of lead system 208, and includes, by way of example and not limitation, implantable leads 408A and 408B. As illustrated in FIG. 4A, the IPG 404 may be coupled to the implantable leads 408A-B at a proximal end of each lead. The distal end of each lead includes electrical contacts or electrodes 406 for contacting a tissue site targeted for electrical neurostimulation. By way of example and not limitation, the leads 408A-B each include eight electrodes 406 at the distal end. Other numbers and arrangements of leads 408A-B and electrodes 406 may also be used. In various examples, one or more of the electrodes 406 may be column electrodes (or ring electrodes), or segmented electrodes circumferentially disposed on a directional lead such as 408A or 408B.

The implantable leads and electrodes may be shaped and sized to provide electrical neurostimulation energy to a neural target, such as a brain, a nerve target of a spinal cord, or a peripheral nerve target. Neurostimulation energy may be delivered in a unipolar mode between an electrode selected from electrodes 406 and another electrode selected from electrodes 426 and 428, or in a balanced or unbalanced bipolar mode using a pair, or more, of electrodes on the same lead (e.g., lead 408A or lead 408B), with the balancing current being applied to reference electrodes 426 or 428. Neurostimulation energy may be delivered in an extended bipolar mode using one or more electrodes of a lead (e.g., one or more electrodes of lead 408A) and one or more electrodes of a different lead (e.g., one or more electrodes of lead 408B).

The electronic circuitry of IPG 404 may include a control circuit that controls delivery of the neurostimulation energy. The control circuit may include a microprocessor, a digital signal processor, application specific integrated circuit (ASIC), or other type of processor, interpreting or executing instructions included in software or firmware. The neurostimulation energy may be delivered according to specified (e.g., programmed) modulation parameters. Examples of setting modulation parameters may include, among other things, selecting the electrodes or electrode combinations used in the stimulation, configuring an electrode or electrodes as the anode or the cathode for the stimulation, and specifying stimulation pulse parameters. Examples of pulse parameters include, among other things, the amplitude of a pulse (specified in current or voltage), pulse duration (e.g., in microseconds), pulse rate (e.g., in pulses per second), and parameters associated with a pulse train or pattern such as burst rate (e.g., an "on" modulation time followed by an "off" modulation time), amplitudes of pulses in the pulse train, polarity of the pulses, etc.

The modulation parameters may additionally include fractionalization across electrodes. The fractionalization specifies distribution (e.g., the percentage) of the stimulation current, voltage, or electrical energy provided by an electrode or electrode combination, which affect the spatial distribution of the resultant stimulation field. In an example, current fractionalization specifies percentage cathodic current, percentage anodic current, or off (no current allocation). FIG. 4B illustrates a segment of an electrical neuromodulation lead that includes multiple electrodes 416, which may be an embodiment of the electrodes 406 of the lead 408A or 408B. Current is fractionalized across the active electrodes, and the electrodes 416 each may receive a respective current percentage. In the monopolar case, the fractionalized currents across the active electrodes add up to 100%. In the bipolar or multipolar cases, the fractionalized currents for at least one polarity add up to 100%, with any remaining percentage being allocated to the reference electrodes. Control of the current in terms of percentage allows precise and consistent distribution of the current among the electrodes even as the current amplitude is adjusted. It is suited for thinking about the problem as steering a stimulation locus, and stimulation changes on multiple contacts simultaneously to move the locus while holding the stimulation amount constant. In some examples, the current fractionalization may be defined by assigning an absolute current value (e.g., in milliampere, or mA) rather than a percentage to each electrode. Control of the current in terms of absolute values allows precise dosing of current through each specific electrode. It is suited for changing the current one contact at a time (and allows the user to do so) to shape the stimulation like a piece of clay (pushing/pulling one spot at a time).

The current fractionalization takes into account electrode / tissue coupling differences, which are the differences in how the tissue underlying each electrode reacts to electrical neuromodulation. In addition, electrodes on the distal portion of the lead may have lower gradient in the longitudinal direction, as electrical field strength may taper down at the ends of the lead. Current fractionalization may accommodate variation in the tissue underlying those electrodes. Various embodiments described herein implement a programmed algorithm to determine the appropriate fractionalization to achieve a desired neuromodulation field property.

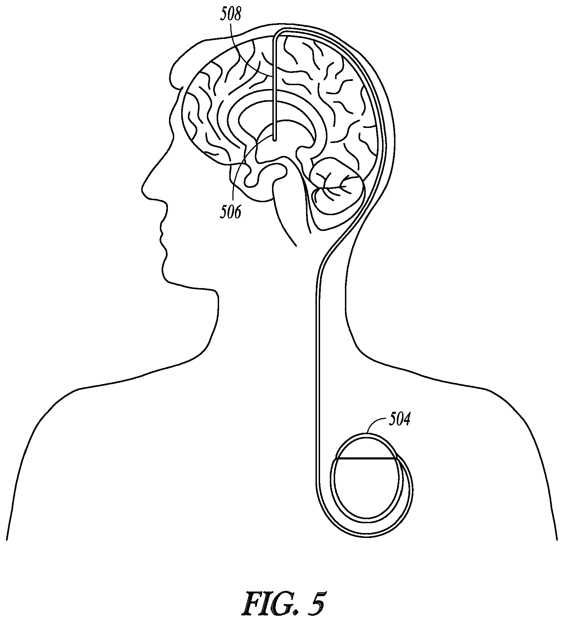

FIG. 5 illustrates, by way of example and not limitation, an embodiment of an IPG 504 and an implantable lead system 508 arranged to provide brain stimulation to a patient. An example of IPG 504 includes the IPG 404. The lead system 508 may include electrodes 506. An example of lead system 508 includes one or more of the leads 408A-B. An example of the electrodes 506 includes at least a portion of the electrodes 406. In the illustrated example, the IPG 504 and the implantable lead system 508 may provide Deep Brain Stimulation (DBS) to a patient, with the stimulation target being neuronal tissue in a subdivision of the thalamus of the patient's brain. Other examples of DBS targets include neuronal tissue of the globus pallidus (GPi), the subthalamic nucleus (STN), the pedunculopontine nucleus (PPN), substantia nigra pars reticulate (SNr), cortex, globus pallidus externus (GPe), medial forebrain bundle (MFB), periaquaductal gray (PAG), periventricular gray (PVG), habenula, subgenual cingulate, ventral intermediate nucleus (VIM), anterior nucleus (AN), other nuclei of the thalamus, zona incerta, ventral capsule, ventral striatum, nucleus accumbens, white matter tracts connecting these and other structures. The DBS targets may also include regions determined analytically based on side effects or benefits observed in one or more patients, as well as regions specified by the user.

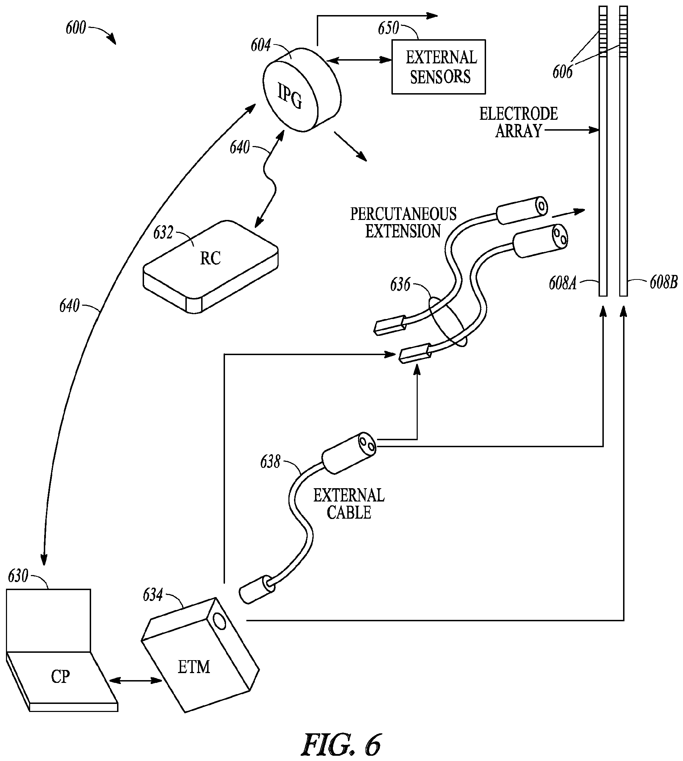

FIG. 6 illustrates, by way of example and not limitation, an embodiment of portions of a neurostimulation system 600. The system 600 includes an IPG 604, implantable neurostimulation leads 608A and 608B, an external remote controller (RC) 632, a clinician's programmer (CP) 630, and an external trial modulator (ETM) 634. The system 600 may additionally include external sensors 650 configured to sense one or more physiological parameters, such as a heart rate sensor, a pulse oximeter, an electrocardiogram sensor, an inertial sensor, or an electroencephalogram sensor, among others. The IPG 604 may be electrically coupled to the leads 608A and 608B directly or through percutaneous extension leads 636. The ETM 634 may be electrically connectable to the leads 608A and 608B via one or both of the percutaneous extension leads 636 and/or the external cable 638. The system 600 represents an embodiment of system 100, with IPG 604 representing an embodiment of the stimulation device 104, electrodes 606 of leads 608A and 608B representing the electrodes 106, and CP 630, RC 632, and the ETM 634 collectively representing the programming device 102.

The ETM 634 may be standalone or incorporated into the CP 630. The ETM 634 may have similar pulse generation circuitry as IPG 604 to deliver neurostimulation energy according to specified modulation parameters as discussed above. In an example, the ETM 634 is an external device and may be used as a preliminary stimulator after leads 408A and 408B have been implanted and used prior to stimulation with IPG 604 to test the patient's responsiveness to the stimulation that is to be provided by IPG 604. An external ETM 634 may be more easily configurable than the IPG 604.

The CP 630 may configure the neurostimulation provided by the ETM 634. If the ETM 634 is not integrated into the CP 630, then the CP 630 may communicate with ETM 634 using a wired connection (e.g., over a USB link) or by wireless telemetry such as using a wireless communication link. The CP 630 may also communicate with IPG 604 using a wireless communication link 640.

An example of wireless telemetry is based on inductive coupling between two closely-placed coils using the mutual inductance between these coils. This type of telemetry is referred to as inductive telemetry or near-field telemetry because the coils must typically be closely situated for obtaining inductively coupled communication. The IPG 604 may include the first coil and a communication circuit. The CP 630 may include or otherwise electrically connected to the second coil such as in the form of a wand that may be place near the IPG 604. Another example of wireless telemetry includes a far-field telemetry link, also referred to as a radio frequency (RF) telemetry link. A far-field, also referred to as the Fraunhofer zone, refers to the zone in which a component of an electromagnetic field produced by the transmitting electromagnetic radiation source decays substantially proportionally to 1/r, where r is the distance between an observation point and the radiation source. Accordingly, far-field refers to the zone outside the boundary of r = λ/2π, where λ is the wavelength of the transmitted electromagnetic energy. In one example, a communication range of an RF telemetry link is at least six feet but may be as long as allowed by the particular communication technology. RF antennas may be included, for example, in the header of the IPG 604 and in the housing of the CP 630, eliminating the need for a wand or other means of inductive coupling. An example is such an RF telemetry link is a Bluetooth^{®} wireless link.

The CP 630 may be used to set modulation parameters for the neurostimulation after the IPG 604 has been implanted. This allows the neurostimulation to be tuned if the requirements for the neurostimulation change after implantation. The CP 630 may also upload information from or download information to the IPG 604.

The RC 632 also communicates with the IPG 604 using a wireless link 340. The RC 632 may be a communication device used by the user or given to the patient. The RC 632 may have reduced programming capability compared to the CP 630. This allows the user or patient to alter the neurostimulation therapy but does not allow the patient full control over the therapy. For example, the patient may be able to increase the amplitude of neurostimulation pulses or change the time that a preprogrammed stimulation pulse train is applied. The RC 632 may be programmed by the CP 630. The CP 630 may communicate with the RC 632 using a wired or wireless communications link. In some embodiments, the CP 630 is able to program the RC 632 when remotely located from the RC 632. In some examples, the RC 632 may download data to and upload data from the IPG 604.

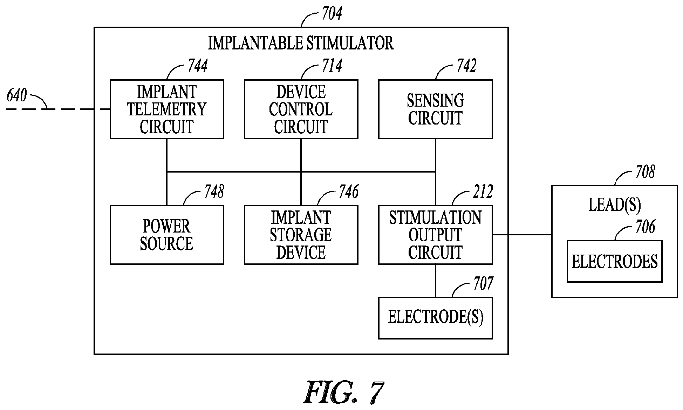

FIG. 7 illustrates, by way of example and not limitation, an embodiment of implantable stimulator 704 and one or more leads 708 of an implantable neurostimulation system, such as the implantable system 600. The implantable stimulator 704 represents an embodiment of stimulation device 104 or 204 and may be implemented, for example, as the IPG 404, 505, or 604. Lead(s) 708 represents an embodiment of lead system 208 and may be implemented, for example, as implantable leads 608A-B. The lead(s) 708 includes electrodes 706, which represents an embodiment of electrodes 106 or 206 and may be implemented as electrodes 606. In some examples, the implantable stimulator 704 may additionally be communicatively coupled to one or more external sensors (such as external sensors 650 in FIG. 6) configured to sense one or more physiological parameters, such as a heart rate sensor, a pulse oximeter, an electrocardiogram sensor, an inertial sensor, or an electroencephalogram sensor, among others.

The implantable stimulator 704 may include a sensing circuit 742 that is optional and required only when the stimulator needs a sensing capability, stimulation output circuit 212, a device control circuit 714, an implant storage device 746, an implant telemetry circuit 744, a power source 748, and one or more electrodes 707. The sensing circuit 742, when included and needed, senses one or more physiologic signals for purposes of patient monitoring and/or feedback control of the neurostimulation. Examples of the physiologic signals include neural and other signals each indicative of a condition of the patient that is treated by the neurostimulation and/or a response of the patient to the delivery of the neurostimulation. The stimulation output circuit 212 is electrically connected to electrodes 706 through one or more leads 708 as well as electrodes 707, and delivers each of the neurostimulation pulses through a set of electrodes selected from electrodes 706 and electrode(s) 707.

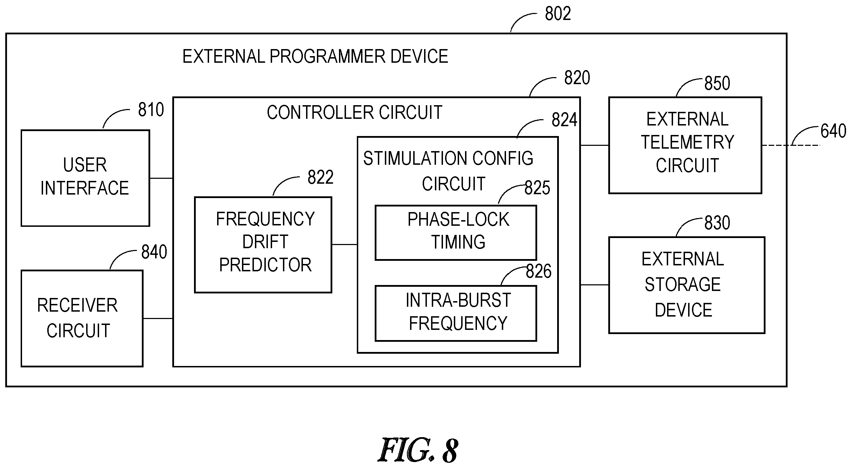

The device control circuit 714 represents an embodiment of device control circuit 214, and controls the delivery of the neurostimulation pulses according to the stimulation configuration (including stimulation parameters) received from the programming device 102 or 302. In an example, the programming device 102 or 302 can determine or adjust a stimulation parameter in the presence of an indication of frequency drift of intrinsic oscillatory neural activity, and the device control circuit 714 can adapt neurostimulation to such frequency drift. Examples of the stimulation parameter may include a burst frequency (e.g., an inter-burst frequency or an intra-burst frequency) and a timing to initiate the burst stimulation at a particular phase of an oscillation cycle of the intrinsic oscillatory neural activity. In an example, the programming device 102 or 302 can determine or adjust the burst stimulation parameter based on patient responses, such as levels of symptom control induced by one or more test stimulation bursts. The device control circuit 714 can control the delivery of neurostimulation in accordance with the determined or adjusted burst stimulation parameter. Examples of adapting neurostimulation to frequency drift of intrinsic oscillatory neural activity are discussed below with reference to FIGS. 8-9.

The implant telemetry circuit 744 provides the implantable stimulator 704 with wireless communication with another device, such as the CP 630 or the RC 632, including receiving values of the plurality of stimulation parameters from the other device. The implant storage device 746 stores the received stimulation configuration, including values of the plurality of stimulation parameters. The power source 748 provides the implantable stimulator 704 with energy for its operation. The power source 748 may include a battery. In one embodiment, the power source 748 includes a rechargeable battery and a battery charging circuit for charging the rechargeable battery. The implant telemetry circuit 744 may also function as a power receiver that receives power transmitted from an external device through an inductive couple. The electrode(s) 707 allow for delivery of the neurostimulation pulses in the monopolar mode or unbalanced bipolar mode. Examples of the electrode(s) 707 include electrode 426 and electrode 418 in IPG 404 as illustrated in FIG. 4A.

In an example, the implantable stimulator 704 may be used as a master database. A patient implanted with implantable stimulator 704 (such as may be implemented as IPG 604) may therefore carry patient information needed for his or her medical care when such information is otherwise unavailable. The implant storage device 746 may be configured to store such patient information. For example, the patient may be given a new RC 632 and/or travel to a new clinic where a new CP 630 is used to communicate with the device implanted in him or her. The new RC 632 and/or CP 630 may communicate with the implantable stimulator 704 to retrieve the patient information stored in implant storage device 746 through the implant telemetry circuit 744 and the wireless communication link 640, and allow for any necessary adjustment of the operation of the implantable stimulator 704 based on the retrieved patient information.

In various examples, the patient information to be stored in the implant storage device 746 may include, for example, positions of lead(s) 708 and electrodes 706 relative to the patient's anatomy (transformation for fusing computerized tomogram (CT) of post-operative lead placement to magnetic resonance imaging (MRI) of the brain), clinical effect data, objective measurements using quantitative assessments of symptoms (e.g., using microelectrode recording, accelerometers, and/or other sensors), and/or other information considered important or useful for providing adequate care for the patient. In various examples, the patient information to be stored in implant storage device 746 may include data transmitted to implantable stimulator 704 for storage as part of the patient information and data acquired by implantable stimulator 704, such as using the sensing circuit 742.

In various examples, the sensing circuit 742 (if included), stimulation output circuit 212, device control circuit 714, implant telemetry circuit 744, implant storage device 746, and power source 748 may be encapsulated in an implantable housing or case, such as the hermetically-sealed IPG case 422. The electrode(s) 707 may be formed or otherwise incorporated onto implantable housing or case. In numerous examples, the lead(s) 708 are implanted such that the electrodes 706 are placed on and/or around one or more targets to which the neurostimulation pulses are to be delivered, while the implantable stimulator 704 is subcutaneously implanted and connected to the lead(s) 708 at the time of implantation.

FIG. 8 illustrates, by way of example and not limitation, an embodiment of an external programming device 802 of an implantable neurostimulation system, such as the system 600. The external programming device 802 represents an embodiment of programming device 102 or 302, and may be implemented, for example, as the CP 630 and/or the RC 632. In the illustrated example, the external programming device 802 includes a user interface 810, a receiver circuit 840, a controller circuit 820, an external storage device 830, and an external telemetry circuit 850.

The user interface 810, which is an embodiment of user interface 310, allows a user to perform various patient monitoring and electrostimulation programming tasks. An example of the user interface 810 includes a graphical user interface. The user interface 810 may include a display screen and a user input device. The display screen can display stimulation configuration, including various stimulation parameters, or virtual electrodes, among other control parameters. In an example, clinical effect information associated with stimulation may be displayed, including information about therapeutic benefits, side effects, or patient responses to stimulation (e.g., levels of symptom control either automatically detected or self-reported by the patient during DBS therapy). The clinical effects of the stimulation may take the form of a numerical score or a graphical representation. The user input device may include one or more of a touchscreen, a keyboard, a keypad, a touchpad, a trackball, a joystick, or a mouse. The user input device allows a user to confirm, reject, or adjust the stimulation configuration. In an example, a user may use the user input device to adjust one or more stimulation parameters, such as pulse amplitude, pulse width, pulse rate, burst frequency, pulse waveform, or timing of stimulation pulses at a particular phase of an oscillation cycle of an intrinsic oscillatory neural activity. In another example, a user may use the user input device to adjust virtual electrode state, such as adjusting one or more steering parameters for steering a virtual electrode. In yet another example, a user may use the user input device to provide virtual electrode steering parameters, or modify a previously generated virtual electrode steering parameters (e.g., longitudinal coordinate and/or angular coordinate of the virtual electrode).

The receiver circuit 840 may receive physiological information of the patient sensed by one or more electrodes or physiological sensors including, for example, a heart rate sensor, an accelerometer, a pulse oximeter, an electrocardiogram (ECG) sensor, an electromyogram (EMG) sensor, inertial sensor, or an electroencephalogram (EEG) sensor array, among others. The physiological sensors may be ambulatory sensors included in or communicatively coupled to an implantable pulse generator such as the implantable stimulator 704, or the IPG 404, 504, or 604. In some examples, the received physiological information may include intra-operative physiological data collected by a sensing device or a patient monitor separate from the implantable pulse generator during an implantation procedure. In some examples, the received physiological information, either collected by the ambulatory sensors or by separate patient monitors, may be used for detecting or predicting a frequency drift of an intrinsic oscillatory neural activity. As to be discussed in the following, the receiver circuit 840 may be coupled to one or more physiological sensors to sense patient responses to electrostimulation indicative of levels of symptom control during a stimulation optimization session. The patient responses may be used to optimize stimulation parameters.

The controller circuit 820 represents an embodiment of stimulation programmer circuit 320, and may generate a stimulation configuration (e.g., a plurality of stimulation parameters) to be transmitted to the implantable stimulator 704. The controller circuit 820 may be a part of external control circuitry in the external programming device 802, and implemented as a part of a microprocessor circuit, which may be a dedicated processor such as a digital signal processor, application specific integrated circuit (ASIC), microprocessor, or other type of processor for processing information including physical activity information. Alternatively, the microprocessor circuit may be a general-purpose processor that may receive and execute a set of instructions of performing the functions, methods, or techniques described herein.

The controller circuit 820 may include circuit sets comprising one or more other circuits or sub-circuits, including a frequency drift predictor 822 and a stimulation configuration circuit 824. These circuits may, alone or in combination, perform the functions, methods, or techniques described herein. In an example, hardware of the circuit set may be immutably designed to carry out a specific operation (e.g., hardwired). In an example, the hardware of the circuit set may include variably connected physical components (e.g., execution units, transistors, simple circuits, etc.) including a computer readable medium physically modified (e.g., magnetically, electrically, moveable placement of invariant massed particles, etc.) to encode instructions of the specific operation. In connecting the physical components, the underlying electrical properties of a hardware constituent are changed, for example, from an insulator to a conductor or vice versa. The instructions enable embedded hardware (e.g., the execution units or a loading mechanism) to create members of the circuit set in hardware via the variable connections to carry out portions of the specific operation when in operation. Accordingly, the computer readable medium is communicatively coupled to the other components of the circuit set member when the device is operating. In an example, any of the physical components may be used in more than one member of more than one circuit set. For example, under operation, execution units may be used in a first circuit of a first circuit set at one point in time and reused by a second circuit in the first circuit set, or by a third circuit in a second circuit set at a different time.

The frequency drift predictor 822 can predict frequency drift of an intrinsic oscillatory neural activity, or an indication thereof, using physiological information received from the receiver circuit 840. The physiological information can include ambulatory physiological data collected by one or more sensors included in or communicatively coupled to the implantable stimulator 704, such as an accelerometer or ECG sensor. Additionally or alternatively, the physiological information can include intra-operative physiological data collected by patient monitoring devices separate from the implantable stimulator 704 during an implantation procedure.

In some examples, the frequency drift predictor 822 can predict the indication of the frequency drift of the intrinsic oscillatory neural activity based at least on patient responses to burst stimulation delivered to a neural target (e.g., a brain target) in accordance with a stimulation parameter. Evaluation of the patient responses and prediction of frequency drift may be carried out periodically or at scheduled intervals. To evaluate the patient responses to stimulation, in an example, a plurality of stimulation bursts may be delivered to the neural target in accordance with their respective burst stimulation parameter values. The patient responses may be evaluated using physiological signals acquired by a wearable device, such as a device with wearable sensors for sensing physiological signals indicative of therapeutic effect, side effects, or symptom relief induced by the test stimulation bursts. Additionally or alternatively, a user (e.g., the patient or a care provider) may report patient responses such as via a user interface device (e.g., the CP 630, the RC 632, a smartphone, or other personal electronic devices).

As discussed above, stimulation bursts arriving at specific phase(s) of an oscillation cycle of an intrinsic oscillatory neural activity in the patient may be more effective than bursts arriving at other phases of the oscillation cycle. A frequency drift may cause misalignment or loss of synchronization between the stimulation burst and optimal phase of the intrinsic oscillatory neural activity. As such, worsening symptoms or reduced symptom relief, and/or reduced stimulation efficacy, may be indicative of a newly developed frequency drift of the intrinsic oscillatory neural activity.

The frequency drift predictor 822 can perform frequency analysis of the received physiological information and determine a dominant frequency within a specific frequency band (e.g., below 10 Hz). Such dominant frequency corresponds to an intrinsic oscillatory activity, such as essential tremor. In another example, the frequency drift predictor 822 can generate a trend of a signal metric of the received physiological information over time, and predict the frequency drift using the signal metric trend. An example of the signal metric includes a cycle length of the intrinsic oscillatory neural activity.

In addition or alternative to automatic detection or prediction of a frequency drift by the frequency drift predictor 822, an indication of frequency drift may be determined by a separate device other than the external programmer device 802, such as a patient monitor during a procedure of implanting an implantable pulse generator. Such frequency drift indication may be provided to the receiver circuit 840. In some examples, a user may provide an indication of frequency drift to the external programmer device 802, such as via the user interface 810, or other user interface devices in communication with the external programmer device 802.

The indication of frequency drift may be presented to a user (e.g., a clinician), or be provided to a process to guide adjustment or optimization of a burst stimulation parameter. In the presence of frequency drift of an intrinsic oscillatory neural activity, burst stimulation parameters (e.g., phase-lock timing and/or burst frequency) may be sub-optimal, thereby reducing the therapeutic effectiveness of motor control. The patient may demonstrate more prominent tremor, rigidity, or dystonia symptoms. The stimulation configuration circuit 824 may determine or adjust one or more stimulation parameters in response to an indication of frequency drift of the intrinsic oscillatory neural activity such as predicted by the frequency drift predictor 822 or provided by the user. In a non-limiting example as shown in FIG. 8, the stimulation parameters to be determined or adjusted can include one or more of a phase-lock timing 825 or a burst frequency 826. The phase-lock timing 825 represents the timing to initiate burst stimulation at a particular phase of an oscillation cycle of an intrinsic oscillatory neural activity. Burst frequency 826 can include, for example, an inter-burst frequency or an intra-burst frequency. Stimulation bursts arriving at specific phases of an intrinsic oscillation may be more effective than ones that arrive at other times. For example, timing the bursts to coincide with a trough in an oscillation cycle can produce more effective symptom control (e.g., more significant symptom relief) than timing the bursts to coincide with a peak in the oscillation cycle. The stimulation configuration circuit 824 can detect a specific phase of the intrinsic oscillation cycle, such as a trough or substantially close thereto (e.g., within a specified tolerance), and time the burst stimulation to coincide with the detected phase.

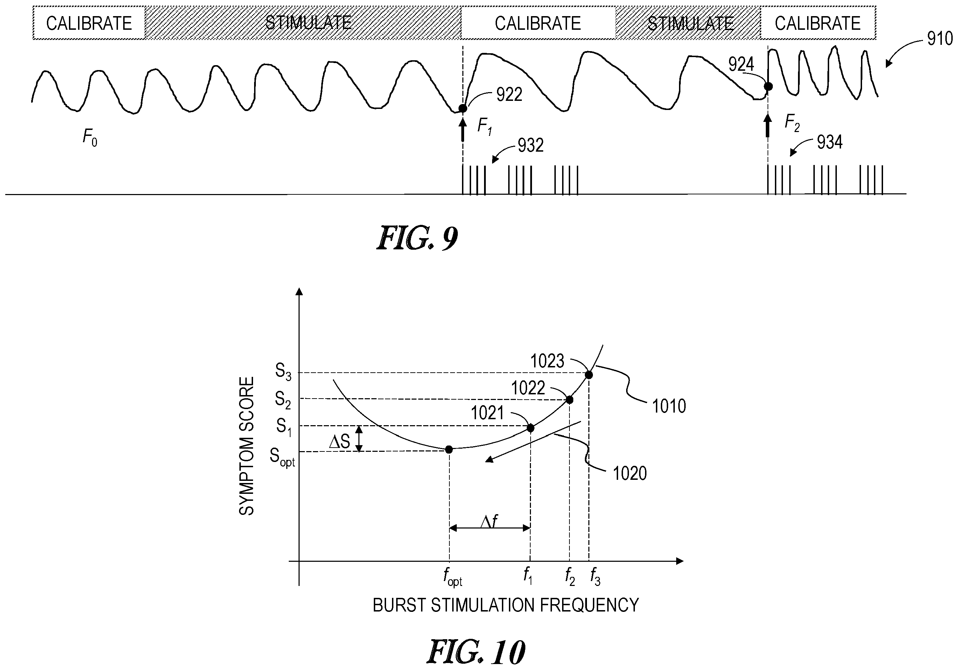

In some examples, the stimulation configuration circuit 824 can additionally or alternatively determine or adjust the burst frequency 826 in response to the detected indication of frequency drift. The burst frequency 826 can be adjusted in an increasing direction or a decreasing direction. In an example, the burst frequency 826 can be increased in response to an increasing trend of frequency drift, or be decreased in response to a decreasing trend of the frequency drift. Examples of determining a target or optimal burst frequency in the presence of frequency drift of an intrinsic oscillatory neural activity is discussed below with reference to FIG. 10.

In various examples, the stimulation configuration circuit 824 may adjust one or more stimulation parameters at periodic intervals, such as every 1-3 days, every week, every other week, or at other specific intervals. In an example, the stimulation configuration circuit 824 may adjust one or more stimulation parameters at a specific time of a day. In an example, the stimulation configuration circuit 824 may adjust one or more stimulation parameters in response to a trigger event which may include, for example, worsening symptoms, a change in health status, a change in medication, receiving a treatment, or a patient state (e.g., sleep or wake).

In some examples, the stimulation configuration circuit 824 may determine or adjust one or more stimulation parameters (e.g., the phase-lock timing 825 and the burst frequency 826) based on patient responses to a plurality of test stimulation bursts delivered during a stimulation optimization session (also referred to as a calibration session). The stimulation optimization session can be triggered by a predicted frequency drift of the intrinsic oscillatory neural activity, and involves sequentially delivering a plurality of test stimulation bursts each having respective stimulation parameter values. In an example, the stimulation optimization session may be initiated on a regular basis automatically, or alternatively be notified to a user (e.g., a clinician or the patient) and commences upon user's confirmation, such as via the user interface 810. During the stimulation optimization session, a plurality of test stimulation bursts can be delivered in accordance with the burst stimulation parameter taking respective values, and the stimulation configuration circuit 824 can evaluate respective patient responses to each of the plurality of test stimulation bursts. The burst stimulation parameter value can vary in a ramp-up or a ramp-down mode, where the stimulation parameter value may be gradually increased (ramping up) or decreased (ramping down). The burst stimulation parameter value can ramp up or ramp down linearly at a specific step size. In a non-limiting example, the burst stimulation frequency can ramp up at 0.1 Hz increment or ramp down at 0.1 Hz decrement. In some examples, a variable or adaptive step size can be used during the ramp-up or ramp-down process. In a non-limiting example, the phase-lock timing can ramp up at 100 milliseconds (msec) increment or ramp down at 100 msec decrement. In an example, the step size can be programmable.

In response to the test stimulation bursts with ramped up or ramped down stimulation parameter values, respective patient responses may be evaluated. In an example, the patient responses may be evaluated using a wearable device, such as a device with wearable sensors for sensing physiological signals indicative of therapeutic effect, side effects, or symptom relief induced by the test stimulation bursts. In another example, the patient responses may be reported by a user (e.g., the patient or a care provider) via a user interface device, such as the CP 630, the RC 632, a smartphone, or other personal electronic devices. The patient responses to test stimulation bursts may be received by the receiver circuit 840.

The patient responses to the test stimulation bursts can include levels of symptom relief or improved motor function control, such as alleviation or reduction in severity and/or duration of tremor, slowness and rigidity, dystonia (involuntary repetitive or twisting movements), or essential tremor, among other symptoms. A target or optimal stimulation parameter may be searched through an optimization process. In an example, the stimulation configuration circuit 824 can determine a target burst stimulation parameter value as one corresponding to a greater symptom relief than stimulation bursts having other one or more stimulation parameter values. In an example where the plurality of test stimulation bursts are delivered in accordance with respective burst frequencies, the stimulation configuration circuit 824 can evaluate respective levels of symptom relief, and determine a target burst frequency to be one that induces a greater symptom relief than stimulation bursts having other one or more burst frequencies. In an example, the target or optimal burst frequency can be determined to be the one associated with the greatest level of symptom relief (or the lowest symptom score) among the tested burst frequencies. In another example where the plurality of test stimulation bursts are synchronized with respective phases of the oscillation cycle of the intrinsic oscillatory neural activity, the stimulation configuration circuit 824 can evaluate respective levels of symptom relief, and determine a target phase of an oscillation cycle for timing a burst stimulation to be one that induces a greater symptom relief than stimulation bursts timed at other one or more phases of the oscillation cycle. In an example, the target or optimal phase can be determined to be the one associated with the greatest level of symptom relief (or the lowest symptom score) among the tested phases.

As an alternative to searching a target or optimal stimulation parameter from the tested stimulation parameter values in an optimization session, in some examples, the stimulation configuration circuit 824 can generate a parameter prediction model using the tested stimulation parameter values and respective patient response (e.g., levels of symptom relief). The parameter prediction model represents a linear or nonlinear relationship between the stimulation parameter values and the level of symptom relief induced by the test stimulation bursts. Examples of a parameter prediction model and using the same to search for a target or optimal stimulation parameter are discussed below with reference to FIG. 10.

The external telemetry circuit 850 provides the external programming device 802 with communication with another device, such as the implantable stimulator 704 via the wireless communication link 640. The communication between the external programming device 802 and the implantable stimulator 704 may include transmission of the stimulation configuration to the implantable stimulator 704, including the phase-lock timing 825 and the burst frequency 826, among other parameters that are determined in a stimulation optimization session. Data transmission between the external programming device 802 and the implantable stimulator 704 may be continuous, periodic at a specified period, or triggered by a user command or a specific event. In one embodiment, the external telemetry circuit 850 may also transmit power to the implantable stimulator 704 through a charging circuit such as an inductive couple.

In various examples, the wireless communication link 640 may include an inductive telemetry link (near-field telemetry link) and/or a far-field telemetry link (RF telemetry link). For example, because DBS is often indicated for movement disorders that are assessed through patient activities, gait, balance, etc., allowing patient mobility during programming and assessment is useful. Therefore, when the system 600 is intended for applications including DBS, the wireless communication link 640 may include at least a far-field telemetry link, either near field or far field, that allows for communications between the external programming device 802 and the implantable stimulator 704 over a relative long distance, such as up to about 20 meters. In various examples, the external telemetry circuit 850 and implant telemetry circuit 744 each may include an antenna and RF circuitry configured to support such wireless telemetry.

The external storage device 830 may store the stimulation configuration, including the phase-lock timing 825, the burst frequency 826 among other stimulation parameters and building blocks for defining one or more stimulation waveforms. The one or more stimulation waveforms may each be associated with one or more stimulation fields and represent a pattern of neurostimulation pulses to be delivered to the one or more stimulation field during the neurostimulation therapy session. In various examples, each of the one or more stimulation waveforms may be selected for modification by the user and/or for use in programming a stimulation device such as the implantable stimulator 704 to deliver a therapy. In various examples, each waveform in the one or more stimulation waveforms is definable on a pulse-by-pulse basis, and the external storage device 830 may include a pulse library that stores one or more individually definable pulse waveforms each defining a pulse type of one or more pulse types. The external storage device 830 may also store one or more individually definable stimulation fields. Each waveform in the one or more stimulation waveforms is associated with at least one field of the one or more individually definable stimulation fields. Each field of the one or more individually definable stimulation fields is defined by a set of electrodes through a neurostimulation pulse is delivered. In various examples, each field of the one or more individually definable fields is defined by the set of electrodes through which the neurostimulation pulse is delivered and a current distribution of the neurostimulation pulse over the set of electrodes. In an example, a parameter prediction model generated using test stimulation bursts and patient symptoms during a stimulation optimization session, such as that illustrated in FIG. 10, can also be stored in the external storage device 830.

The stimulation configuration, including the including the phase-lock timing 825, the burst frequency 826, among other stimulation parameters, may be displayed to a user via the user interface 810. Information about the frequency drift, such as that predicted by the frequency drift predictor 822, may also be displayed to a user via the user interface 810. In an example, a user may adjust the stimulation configuration using a user input device through a GUI of the user interface 810. The adjusted stimulation configuration may be stored in the external storage device 830. In various examples, the controller circuit 820 may check values of the plurality of stimulation parameters against safety rules to limit these values within constraints of the safety rules. In one embodiment, the safety rules are heuristic rules.

FIG. 9 is a diagram illustrating an example of frequency drift of an intrinsic oscillatory neural activity 910 and calibration of a burst stimulation parameter for DBS therapy, such as a phase-lock timing in this example. The intrinsic oscillatory neural activity 910 can be detected using an accelerometer, and is reflective of an underlying pathological condition such as essential tremor. In the illustrated example, the intrinsic oscillation frequency changes from a baseline frequency F₀ to a lower frequency F₁. The frequency drift from F₀ to F₁ can be detected by the frequency drift predictor 822. Due to the frequency drift, the existing DBS therapy (burst stimulation) may be less effective, and the patient may experience worsening symptoms indicating reduced DBS efficacy. The worsening symptoms can be detected automatically by a wearable device, or reported by the patient via a user interface device as discussed above with reference to FIG. 8. The detected frequency drift or the worsening symptoms can trigger a stimulation optimization session (also referred to as a calibration session) to determine a target or optimal stimulation parameter value, such as an optimal phase-lock timing 922 and/or an optimal burst frequency of stimulation burst 932. In the illustrated example, the optimal phase-lock timing 922 can be determined to be a trough (or substantially close thereto within a specified tolerance) of the oscillation cycle of the intrinsic oscillatory neural activity. Additionally, the optimal burst frequency of stimulation burst 932 can be determined based on measurements in the stimulation optimization session, including respective burst stimulation frequencies of a plurality of test stimulation bursts and quantitative measurements of symptoms. In an example, the optimal burst frequency can be determined using a parameter prediction model, as discussed with reference to FIG. 10.

Prediction of frequency drift can be attempted regularly at specified times, or triggered by events such as worsening symptoms. When the intrinsic oscillation frequency changes to a different frequency F₂, another stimulation optimization session may be activated to determine target or optimal stimulation parameter values in accordance with the oscillation frequency F₂, such as an optimal phase-lock timing 924 and/or an optimal burst frequency of stimulation burst 934. In some examples, a stored parameter prediction model may be used to quickly determine optimal stimulation parameter values, as discussed in the following with reference to FIG. 10.

FIG. 10 is a diagram illustrating an example of a parameter prediction model 1010 representing a relationship between stimulation parameter values (e.g., burst stimulation frequencies in this example, on the x-axis) and quantitative measures of symptoms (e.g., symptom scores, on the y-axis) induced by stimulation bursts having the respective stimulation parameter values. The parameter prediction model 1010 may be generated using a plurality of test burst stimulation frequencies { *f*₁, *f*₂, *f*₃, ....}and the corresponding symptom scores {S₁, S₂, S₃, ... } obtained from a parameter optimization session as discussed above. The symptom scores can take numerical values (e.g., between 1-5) indicating different symptom severity levels, such that a smaller symptom value indicates more significant relief and thus a higher therapy efficacy. In an example, the symptom scores can be a composite score computed using a weighted combination of symptom scores respectively determined for a plurality of symptoms.

In an example, the parameter prediction model 1010 can be generated by fitting the burst stimulation frequency-symptom score data pairs, represented in FIG. 10 as test data points (*f*₁, S₁) 1011, (*f*₂, S₂) 1012, (*f*₃, S₃) 1013, ..., into a curve (as shown in FIG. 10) or other parametric or non-parametric models. Since the test burst stimulation frequencies{ *f*₁, *f*₂, *f*₃, ... } may not include an optimal stimulation frequency *f*ₒₚₜ of a stimulation burst capable of inducing a minimal symptom score Sₒₚₜ (representing the most significant symptom relief), the stimulation configuration circuit 824 may search for *f*ₒₚₜ using an optimization process based on the parameter prediction model 1010. In an example, the stimulation configuration circuit 824 may determine an optimal direction 1020 for adjusting the burst frequency based on the test data 1011, 1012, 1013, ..., etc. The optimal direction 1020 may help expedite the process of searching for optimal burst stimulation frequency *f*ₒₚₜ, such as by using a gradient descent algorithm. The optimal burst stimulation frequency*f*ₒₚₜ may be transmitted to an implantable stimulator via the external telemetry circuit. 850, and the implantable stimulator can generate burst stimulation in accordance with *f*ₒₚₜ, along with other optimized stimulation parameters.

The parameter prediction model 1010 can be individualized for each patient. The parameter prediction model 1010 can be updated regularly, upon user request, or in response to a trigger event such as a change in patient health condition. The parameter prediction model 1010 can be stored in the external storage device 830 for future use. For example, while a patient is being treated with stimulation bursts having a burst frequency *f*, if the patient's symptoms worsen such that the symptom score increases (from the optimal symptom Sₒₚₜ) by a specific amount ΔS, then a desired amount of change in burst stimulation frequency Δ*f* (from the optimal burst frequency *f*ₒₚₜ) can be determined from the parameter prediction model 1010, and the burst stimulation frequency can be increased from*f* to *f +*Δ*f.*

The parameter prediction model 1010 represents a relationship between burst stimulation frequencies (*f*) and patient symptom scores (S). Similar parameter prediction models may be generated for other stimulation parameters (or a combination of stimulation parameters) as a function of patient symptom scores. In an example, the stimulation parameter may include a stimulation schedule that defines a stimulation program used for treating a neural target for a specified period of time. A parameter prediction model similar to the model 1010 may be generated that represents a relationship between different stimulation schedules (e.g., different stimulation programs and/or different time periods of treatment) and corresponding patient symptom scores.

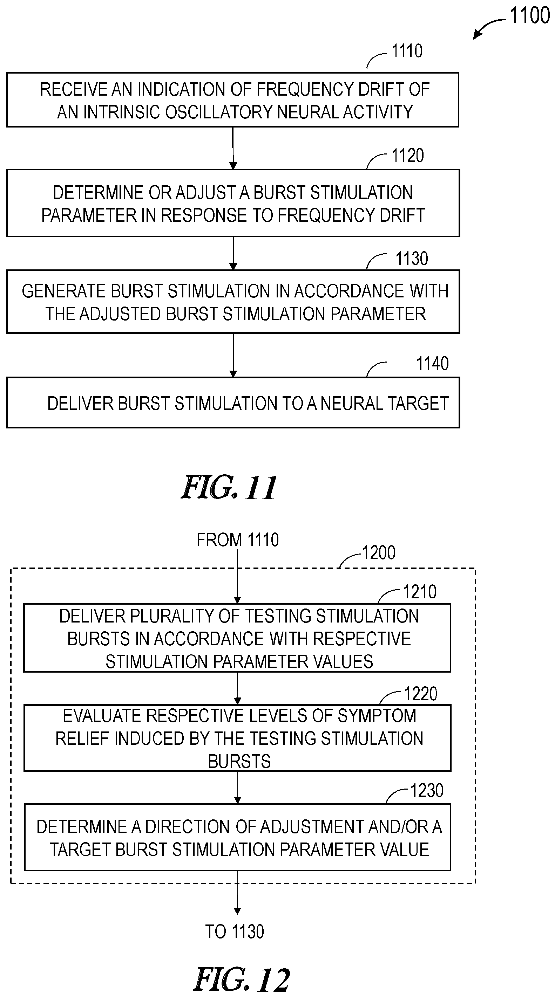

FIG. 11 illustrates, by way of example and not limitation, a non-claimed method 1100 for providing electrostimulation energy to a patient, such as various types of neurostimulation including DBS, in the presence of frequency drift of an intrinsic oscillatory neural activity. The electrostimulation energy can be in a form of stimulation bursts comprising a pulse train followed by a pulse-free period. The stimulation bursts may be delivered in adaptation to frequency drift of an intrinsic oscillatory neural activity. The method 1100 may be implemented in a neurostimulation system, such as system 600 of FIG. 6. Portions of the method 1100 may be implemented in and executed by one of the external programming devices 102, 302, or 802, and used to program an implantable device, such as the stimulator 704 or one of the IPGs 404, 504, or 604. In an example, at least a portion of the method 1100 may be implemented and executed by the CP 630 and/or the RC 632.

The method 1100 begins at step 1110, where an indication of frequency drift of an intrinsic oscillatory neural activity may be received. The intrinsic oscillatory neural activity may be caused by patient underlying pathological conditions, such as essential tremor or neurodegenerative diseases such as Parkinson's disease, and may exhibit slow frequency drift in a range of, for example, 5 Hz to 6 Hz over a few minutes. In an example, the indication of frequency drift may be provided by a user. In another example, the frequency draft may be detected using physiological information sensed from the patient, such as physiological data collected by ambulatory sensors included in or communicatively coupled to an ambulatory stimulator, or intra-operative physiological data collected during an implantation procedure by patient monitoring devices separate from the implantable neurostimulator. The frequency drift may be detected using frequency analysis of the received physiological information, or by trending a signal metric of a received physiological signal over time. In yet another example, the indication of frequency drift may be determined based on patient responses to burst stimulation delivered to a neural target (e.g., a brain target) in accordance with a stimulation parameter. The patient responses may be evaluated using physiological signals acquired by a wearable device, or be reported by the user via a user interface device. Worsening symptoms (or reduced symptom relief), and/or reduced stimulation efficacy, may be indicative of a newly developed frequency drift of the intrinsic oscillatory neural activity.

At 1120, a burst stimulation parameter may be determined or adjusted in response to the detected indication of frequency drift, such as using the stimulation configuration circuit 824 of FIG. 8. Examples of the stimulation parameters to be determined or adjusted can include a phase-lock timing and a burst frequency. The phase-lock timing represents the timing to initiate burst stimulation at a particular phase of an oscillation cycle of an intrinsic oscillatory neural activity. Bursts arriving at specific phases of an intrinsic oscillation of neural activity may be more effective than ones that arrive at other times. In an example, a specific phase of an oscillation cycle of the intrinsic oscillatory neural activity, such as trough or substantially close thereto (e.g., within a specified tolerance), can be identified, and stimulation bursts can be timed to initiate at the identified phase. The burst frequency can be adjusted in a specific direction based on the frequency drift. In an example, the burst frequency can be increased in response to an increasing trend of frequency drift, or be decreased in response to a decreasing trend of the frequency drift. In some examples, the stimulation parameters may be determined or adjusted based on respective patient responses (e.g., levels of symptom relief) to each of a plurality of test stimulation bursts delivered during a stimulation optimization session, an example of which is discussed below with reference to FIG. 12.

In various examples, adjustment of the stimulation parameters may be carried out at periodic intervals, at a specific time of a day, or be triggered by one or more events such as worsening symptoms, a change in medical condition of the patient, such as a change in medication or receiving a treatment.

At 1130, burst stimulation can be generated in accordance with the determined or adjusted burst stimulation parameter which may include, for example, a target or optimal timing for initiating the burst stimulation at a particular phase of an oscillation cycle of the intrinsic oscillatory neural activity, or a target or optimal burst frequency, among other stimulation parameters. The burst stimulation can be generated by an implantable neurostimulator. In an example, the determined or adjusted burst stimulation parameter may be stored in a memory device. The determined or adjusted burst stimulation parameter may additionally or alternatively be presented to the user such as via a user interface, and a user may accept, reject, or make further adjustment of the burst stimulation parameter before it is used to generate burst stimulation.

At 1140, the burst stimulation may be delivered to a neural target of the patient. In an example, the burst stimulation may be delivered to one or more DBS targets of brain, such as via a lead system as described above with reference to FIGS. 5-6, to treat or control symptoms of various neurodegenerative diseases such as essential tremor, Parkinson's disease, primary and secondary dystonia, among others.

FIG. 12 illustrates, by way of example and not limitation, a non-claimed method 1200 for determining or adjusting a stimulation parameter based on patient responses to a plurality of test stimulation bursts delivered to the patient in a stimulation optimization session. The method 1200 can be an example of step 1120 of method 1100 for determining or adjusting a burst stimulation parameter, and can be implemented in and executed by, for example, the stimulation configuration circuit 824 as show in FIG. 8. The stimulation optimization session may be initiated automatically on a regular basis, or alternatively be notified to a user (e.g., a clinician or the patient) and commences upon user's confirmation.

In response to an indication of frequency drift of an intrinsic oscillatory neural activity such as detected at 1110, a plurality of test stimulation bursts may be delivered sequentially to the patient at 1210. The test stimulation bursts may each have respective values of a stimulation parameter, such as respective burst frequencies or respective phase-lock timings. The burst stimulation parameter value can be varied in a ramp-up or a ramp-down mode, where the stimulation parameter value may be gradually increased (ramping up) or decreased (ramping down). The burst stimulation parameter value can ramp up or ramp down linearly at a specific step size. In an example, the step size can be programmable.

At 1220, respective patient responses to each of a plurality of test stimulation bursts may be evaluated. In an example, the patient responses may be evaluated using a wearable device, such as a device with wearable sensors for sensing physiological signals indicative of therapeutic effect, side effects, or symptom relief caused by the stimulation. In another example, the patient responses may be reported by a user via a user interface device. The patient responses to test stimulation bursts can include levels of symptom relief or improved motor function control, such as alleviation or reduction in severity and/or duration of tremor, slowness and rigidity, dystonia (involuntary repetitive or twisting movements), or essential tremor. In an example, numerical symptom scores can be generated to qualify severities of one or more symptoms induced by the test stimulation bursts each having respective stimulation parameter values.

At 1230, a direction of adjustment of stimulation parameter and/or a target or optimal burst stimulation parameter value, can be determined based on the levels of symptom relief obtained at 1220. The target or optimal stimulation parameter may be searched through an optimization process. In an example, the target or optimal burst stimulation parameter value can be determined as one having a greater symptom relief than other one or more burst stimulation parameter values. In an example where the plurality of test stimulation bursts are delivered in accordance with respective burst frequencies, a target or optimal burst frequency may be determined as one that induces a greater symptom relief than other one or more burst frequencies. In an example, the target or optimal burst frequency can be determined to be the one that induces the greatest amount of symptom relief (or the lowest symptom score) among the tested burst frequencies. In another example where the plurality of test stimulation bursts are synchronized with respective phases of the oscillation cycle of the intrinsic oscillatory neural activity, a target or optimal phase of. an oscillation cycle to time a burst stimulation to be one that produces a greater level of symptom relief than stimulation bursts timed at other one or more phases of the oscillation cycle. In an example, the target or optimal phase can be determined to be the one that corresponds to the greatest amount of symptom relief (or the lowest symptom score) among the tested phases.

In an example, a parameter prediction model representing a relationship between stimulation parameter values and symptom levels induced by stimulation bursts having the respective stimulation parameter values can be established, as discussed above with reference to FIG. 10. The parameter prediction model may be used to determine an optimal direction for adjusting a stimulation parameter (e.g., burst frequency). The prediction model may also be used to determine a target or optimal stimulation parameter, such as by using a gradient descent algorithm, as described above with reference to FIG. 10. The parameter prediction model can be stored in a storage device for future use. The target or optimal burst stimulation parameters, such as the burst frequency and the phase-lock timing, may be used for generating the burst stimulation at 1130.

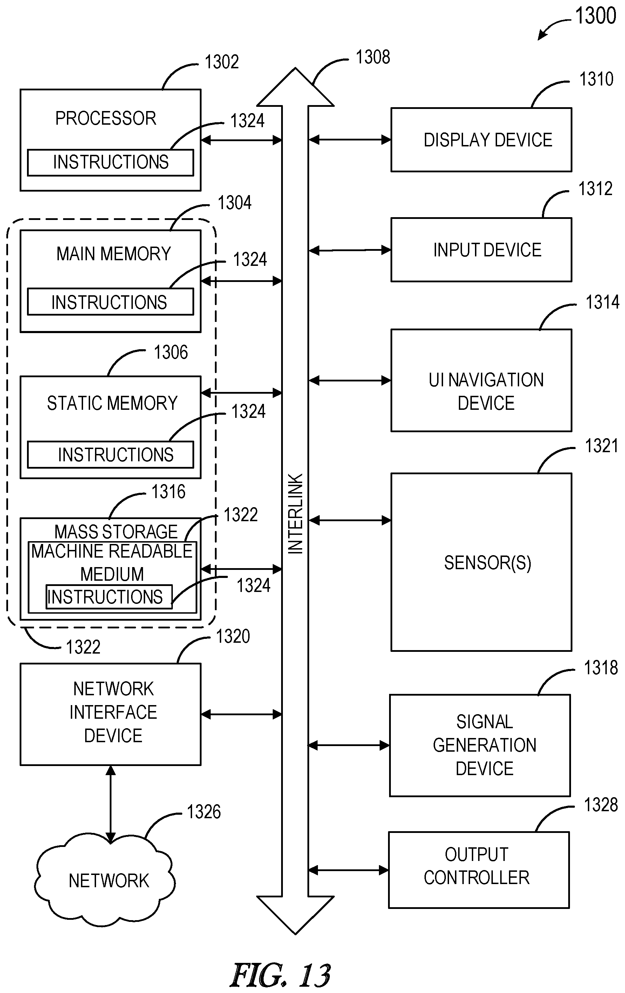

FIG. 13 illustrates generally a block diagram of an example machine 1300 upon which any one or more of the techniques (e.g., methodologies) discussed herein may perform. Portions of this description may apply to the computing framework of various portions of the external programmer device 802.

In alternative embodiments, the machine 1300 may operate as a standalone device or may be connected (e.g., networked) to other machines. In a networked deployment, the machine 1300 may operate in the capacity of a server machine, a client machine, or both in server-client network environments. In an example, the machine 1300 may act as a peer machine in peer-to-peer (P2P) (or other distributed) network environment. The machine 1300 may be a personal computer (PC), a tablet PC, a set-top box (STB), a personal digital assistant (PDA), a mobile telephone, a web appliance, a network router, switch or bridge, or any machine capable of executing instructions (sequential or otherwise) that specify actions to be taken by that machine. Further, while only a single machine is illustrated, the term "machine" shall also be taken to include any collection of machines that individually or jointly execute a set (or multiple sets) of instructions to perform any one or more of the methodologies discussed herein, such as cloud computing, software as a service (SaaS), other computer cluster configurations.

Examples, as described herein, may include, or may operate by, logic or a number of components, or mechanisms. Circuit sets are a collection of circuits implemented in tangible entities that include hardware (e.g., simple circuits, gates, logic, etc.). Circuit set membership may be flexible over time and underlying hardware variability. Circuit sets include members that may, alone or in combination, perform specific operations when operating. In an example, hardware of the circuit set may be immutably designed to carry out a specific operation (e.g., hardwired). In an example, the hardware of the circuit set may include variably connected physical components (e.g., execution units, transistors, simple circuits, etc.) including a computer readable medium physically modified (e.g., magnetically, electrically, moveable placement of invariant massed particles, etc.) to encode instructions of the specific operation. In connecting the physical components, the underlying electrical properties of a hardware constituent are changed, for example, from an insulator to a conductor or vice versa. The instructions enable embedded hardware (e.g., the execution units or a loading mechanism) to create members of the circuit set in hardware via the variable connections to carry out portions of the specific operation when in operation. Accordingly, the computer readable medium is communicatively coupled to the other components of the circuit set member when the device is operating. In an example, any of the physical components may be used in more than one member of more than one circuit set. For example, under operation, execution units may be used in a first circuit of a first circuit set at one point in time and reused by a second circuit in the first circuit set, or by a third circuit in a second circuit set at a different time.

Machine (e.g., computer system) 1300 may include a hardware processor 1302 (e.g., a central processing unit (CPU), a graphics processing unit (GPU), a hardware processor core, or any combination thereof), a main memory 1304 and a static memory 1306, some or all of which may communicate with each other via an interlink (e.g., bus) 1308. The machine 1300 may further include a display unit 1310 (e.g., a raster display, vector display, holographic display, etc.), an alphanumeric input device 1312 (e.g., a keyboard), and a user interface (UI) navigation device 1314 (e.g., a mouse). In an example, the display unit 1310, input device 1312 and UI navigation device 1314 may be a touch screen display. The machine 1300 may additionally include a storage device (e.g., drive unit) 1316, a signal generation device 1318 (e.g., a speaker), a network interface device 1320, and one or more sensors 1321, such as a global positioning system (GPS) sensor, compass, accelerometer, or other sensors. The machine 1300 may include an output controller 1328, such as a serial (e.g., universal serial bus (USB), parallel, or other wired or wireless (e.g., infrared (IR), near field communication (NFC), etc.) connection to communicate or control one or more peripheral devices (e.g., a printer, card reader, etc.).

The storage device 1316 may include a machine readable medium 1322 on which is stored one or more sets of data structures or instructions 1324 (e.g., software) embodying or utilized by any one or more of the techniques or functions described herein. The instructions 1324 may also reside, completely or at least partially, within the main memory 1304, within static memory 1306, or within the hardware processor 1302 during execution thereof by the machine 1300. In an example, one or any combination of the hardware processor 1302, the main memory 1304, the static memory 1306, or the storage device 1316 may constitute machine readable media.

While the machine readable medium 1322 is illustrated as a single medium, the term "machine readable medium" may include a single medium or multiple media (e.g., a centralized or distributed database, and/or associated caches and servers) configured to store the one or more instructions 1324.

The term "machine readable medium" may include any medium that is capable of storing, encoding, or carrying instructions for execution by the machine 1300 and that cause the machine 1300 to perform any one or more of the techniques of the present disclosure, or that is capable of storing, encoding or carrying data structures used by or associated with such instructions. Non-limiting machine readable medium examples may include solid-state memories, and optical and magnetic media. In an example, a massed machine readable medium comprises a machine readable medium with a plurality of particles having invariant (e.g., rest) mass. Accordingly, massed machine-readable media are not transitory propagating signals. Specific examples of massed machine readable media may include: non-volatile memory, such as semiconductor memory devices (e.g., Electrically Programmable Read-Only Memory (EPROM), Electrically Erasable Programmable Read-Only Memory (EEPROM)) and flash memory devices; magnetic disks, such as internal hard disks and removable disks; magneto-optical disks; and CD-ROM and DVD-ROM disks.

The instructions 1324 may further be transmitted or received over a communications network 1326 using a transmission medium via the network interface device 1320 utilizing any one of a number of transfer protocols (e.g., frame relay, internet protocol (IP), transmission control protocol (TCP), user datagram protocol (UDP), hypertext transfer protocol (HTTP), etc.). Example communication networks may include a local area network (LAN), a wide area network (WAN), a packet data network (e.g., the Internet), mobile telephone networks (e.g., cellular networks), Plain Old Telephone (POTS) networks, and wireless data networks (e.g., Institute of Electrical and Electronics Engineers (IEEE) 802.11 family of standards known as WiFi^{®}, IEEE 802.16 family of standards known as WiMax^{®}), IEEE 802.15.4 family of standards, peer-to-peer (P2P) networks, among others. In an example, the network interface device 1320 may include one or more physical jacks (e.g., Ethernet, coaxial, or phone jacks) or one or more antennas to connect to the communications network 1326. In an example, the network interface device 1320 may include a plurality of antennas to wirelessly communicate using at least one of single-input multiple-output (SIMO), multiple-input multiple-output (MIMO), or multiple-input single-output (MISO) techniques. The term "transmission medium" shall be taken to include any intangible medium that is capable of storing, encoding or carrying instructions for execution by the machine 1300, and includes digital or analog communications signals or other intangible medium to facilitate communication of such software.

Various embodiments are illustrated in the figures above. One or more features from one or more of these embodiments may be combined to form other embodiments.

The method examples described herein may be machine or computer-implemented at least in part. Some examples may include a computer-readable medium or machine-readable medium encoded with instructions operable to configure an electronic device or system to perform methods as described in the above examples. An implementation of such methods may include code, such as microcode, assembly language code, a higher-level language code, or the like. Such code may include computer readable instructions for performing various methods. The code may form portions of computer program products. Further, the code may be tangibly stored on one or more volatile or non-volatile computer-readable media during execution or at other times.

The above detailed description is intended to be illustrative, and not restrictive. The scope of the disclosure should, therefore, be determined with references to the appended claims.

## Claims

1. A system for providing electrostimulation to a patient, comprising:
an implantable stimulator (704) configured to provide burst stimulation to a neural target of the patient, the burst stimulation comprising a pulse train followed by a pulse-free period; and
a controller circuit (820) configured to:
receive an indication of frequency drift of an intrinsic oscillatory neural activity in the patient;
determine or adjust a burst stimulation parameter, including a timing to initiate a burst stimulation at a particular phase of an oscillation cycle of the intrinsic oscillatory neural activity, in response to the received indication of frequency drift; and
generate a control signal to the implantable stimulator (704) to provide burst stimulation in accordance with the determined or adjusted burst stimulation parameter.

2. The system of claim 1, wherein the burst stimulation parameter includes a burst frequency (826).

3. The system of any of claims 1-2, wherein the implantable stimulator (704) includes an implantable deep-brain stimulation (DBS) configured to provide burst stimulation to a brain target of the patient.

4. The system of any of claims 1-3, wherein the controller circuit (820) is configured to detect the frequency drift of the intrinsic oscillatory neural activity using physiological information sensed from the patient.

5. The system of any of claims 1-4, wherein to determine or adjust the burst stimulation parameter, the controller circuit (820) is configured to:
evaluate respective patient responses to each of a plurality of test stimulation bursts delivered in accordance with respective values of the burst stimulation parameter;
determine a direction (1020) of adjustment based on the respective patient responses; and
adjust the burst stimulation parameter in accordance with the determined direction (1020).

6. The system of claim 5, wherein the controller circuit (820) is configured to determine a target burst stimulation parameter value based on the respective patient responses.

7. The system of any of claims 5-6, comprising at least one of a wearable device configured to detect the respective patient responses to each of the plurality of test stimulation bursts, or a user interface device configured to receive user inputs about respective patient responses to each of the plurality of test stimulation bursts.

8. The system of any of claims 5-7, wherein to evaluate the respective patient responses, the controller circuit (820) is configured to ramp up or ramp down values of the burst stimulation parameter, and to control the implantable stimulator (704) to deliver the plurality of test stimulation bursts according to the ramped up or ramped down burst stimulation parameter values.

9. The system of any of claims 5-8, wherein the respective patient responses include respective levels of symptom relief in response to each of the plurality of test stimulation bursts, and
wherein the controller circuit (820) is configured to determine a target burst stimulation parameter value as one that has a greater level of symptom relief than other one or more burst stimulation parameter values.

10. The system of claim 9, wherein the controller circuit (820) is configured to:
generate a parameter prediction model (1010) representing a relationship between the burst stimulation parameter values the respective levels of symptom relief; and
predict the target burst stimulation parameter value using the parameter prediction model (1010).

11. The system of any of claims 9-10, wherein the burst stimulation parameter includes a timing to initiate the burst stimulation at a particular phase of an oscillation cycle of the intrinsic oscillatory neural activity, and the controller circuit (820) is configured to:
evaluate respective levels of symptom relief responsive to the plurality of test stimulation bursts initiated at respective phases of the oscillation cycle; and
determine a target phase of the oscillation cycle to initiate burst stimulation that induces a greater symptom relief than burst stimulation initiated at other one or more phases of the oscillation cycle.

12. The system of any of claims 9-11, wherein the burst stimulation parameter includes a burst frequency (826), and the controller circuit (820) is configured to:
evaluate respective levels of symptom relief responsive to the plurality of test stimulation bursts having respective burst frequencies; and
determine a target burst frequency of burst stimulation that induces a greater symptom relief than burst stimulation having other one or more burst frequencies.

13. A system for providing electrostimulation to a patient, comprising:
an implantable stimulator (704) configured to provide burst stimulation to a neural target of the patient, the burst stimulation comprising a pulse train followed by a pulse-free period; and
a controller circuit (820) configured to:
evaluate patient responses to burst stimulation delivered to the neural target in accordance with a stimulation parameter; and
determine an indication of frequency drift of an intrinsic oscillatory neural activity in the patient based on the patient response.

14. The system of claim 13, wherein the controller circuit (820) is configured to evaluate the patient response and to determine the frequency drift periodically or at scheduled intervals.

15. The system of claim 13, wherein to evaluate the patient response to the burst stimulation, the controller circuit (820) is configured to determine respective levels of symptom relief in response to each of a plurality of test stimulation bursts delivered in accordance with respective values of the burst stimulation parameter.

## Patentansprüche

1. System zur Abgabe von Elektrostimulation an einen Patienten, aufweisend:
einen implantierbaren Stimulator (704), der konfiguriert ist, um Burst-Stimulation an ein neurales Target des Patienten abzugeben, wobei die Burst-Stimulation einen Pulszug gefolgt von einer pulsfreien Zeit aufweist; und
eine Steuerschaltung (820), die konfiguriert ist, um:
einen Hinweis auf Frequenzdrift einer intrinsischen oszillatorischen neuralen Aktivität in dem Patienten zu empfangen;
als Antwort auf den empfangenen Hinweis auf Frequenzdrift einen Burst-Stimulation-Parameter zu bestimmen oder anzupassen, der einen Zeitpunkt zum Initiieren einer Burst-Stimulation in einer speziellen Phase eines Oszillationszyklus der intrinsischen oszillatorischen neuralen Aktivität aufweist; und
ein Steuersignal für den implantierbaren Stimulator (704) zu erzeugen, um Burst-Stimulation gemäß dem bestimmten oder angepassten Burst-Stimulation-Parameter abzugeben.

2. System nach Anspruch 1, wobei der Burst-Stimulation-Parameter eine Burst-Frequenz (826) aufweist.

3. System nach einem der Ansprüche 1 bis 2, wobei der implantierbare Stimulator (704) eine implantierbare Deep-Brain-Stimulation (DBS) aufweist, die konfiguriert ist, um Burst-Stimulation an ein Gehirntarget des Patienten abzugeben.

4. System nach einem der Ansprüche 1 bis 3, wobei die Steuerschaltung (820) konfiguriert ist, um die Frequenzdrift der intrinsischen oszillatorischen neuralen Aktivität unter Verwendung physiologischer Information, die an dem Patienten gemessen wird, zu detektieren.

5. System nach einem der Ansprüche 1 bis 4, wobei zum Bestimmen oder Anpassen des Burst-Stimulation-Parameters die Steuerschaltung (820) konfiguriert ist, um:
jeweilige Patientenreaktionen auf jeden von mehreren Teststimulation-Bursts, die gemäß jeweiligen Werten des Burst-Stimulation-Parameters abgegeben werden, zu evaluieren;
auf Basis der jeweiligen Patientenreaktionen eine Anpassungsrichtung (1020) zu bestimmen; und
den Burst-Stimulation-Parameter gemäß der bestimmten Richtung (1020) anzupassen.

6. System nach Anspruch 5, wobei die Steuerschaltung (820) konfiguriert ist, um einen Burst-Stimulation-Parameter-Zielwert auf Basis der jeweiligen Patientenreaktionen zu bestimmen.

7. System nach einem der Ansprüche 5 bis 6, aufweisend eine tragbare Vorrichtung, die konfiguriert ist, um die jeweiligen Patientenreaktionen auf jeden der mehreren Teststimulation-Bursts zu detektieren, und/oder eine Benutzerschnittstelle, die konfiguriert ist, um Benutzereingaben, die jeweilige Patientenreaktionen auf jeden der mehreren Teststimulation-Bursts betreffen, zu empfangen.

8. System nach einem der Ansprüche 5 bis 7, wobei zum Evaluieren der jeweiligen Patientenreaktionen die Steuerschaltung (820) konfiguriert ist, um Werte des Burst-Stimulation-Parameters zu inkrementieren oder zu dekrementieren und den implantierbaren Stimulator (704) zu steuern, die mehreren Teststimulation-Bursts gemäß den inkrementierten oder dekrementierten Burst-Stimulation-Parameterwerten abzugeben.

9. System nach einem der Ansprüche 5 bis 8, wobei die jeweiligen Patientenreaktionen jeweilige Symptomlinderungsgrade als Reaktion auf jeden der mehreren Teststimulation-Bursts aufweisen, und
wobei die Steuerschaltung (820) konfiguriert ist, um als Burst-Stimulation-Parameter-Zielwert denjenigen (Burst-Stimulation-Parameterwert) zu bestimmen, der einen größeren Symptomlinderungsgrad hat als ein oder mehrere andere Burst-Stimulation-Parameterwerte.

10. System nach Anspruch 9, wobei die Steuerschaltung (820) konfiguriert ist, um:
ein Parametervorhersagemodell (1010), das eine Beziehung zwischen den Burst-Stimulation-Parameterwerten und den jeweiligen Symptomlinderungsgraden repräsentiert, zu erzeugen; und
den Burst-Stimulation-Parameter-Zielwert unter Verwendung des Parametervorhersagemodells (1010) vorherzusagen.

11. System nach einem der Ansprüche 9 bis 10, wobei der Burst-Stimulation-Parameter einen Zeitpunkt zum Initiieren der Burst-Stimulation in einer speziellen Phase eines Oszillationszyklus der intrinsischen oszillatorischen neuralen Aktivität aufweist, und die Steuerschaltung (820) konfiguriert ist, um:
jeweilige Symptomlinderungsgrade als Reaktion auf die mehreren Teststimulation-Bursts, die in jeweiligen Phasen des Oszillationszyklus initiiert worden sind, zu evaluieren; und
eine Zielphase des Oszillationszyklus zu bestimmen, um Burst-Stimulation zu initiieren, die eine größere Symptomlinderung induziert als eine in einer oder mehreren anderen Phasen des Oszillationszyklus initiierte Burst-Stimulation.

12. System nach einem der Ansprüche 9 bis 11, wobei der Burst-Stimulation-Parameter eine Burst-Frequenz (826) aufweist, und die Steuerschaltung (820) konfiguriert ist, um:
jeweilige Symptomlinderungsgrade als Reaktion auf die mehreren Teststimulation-Bursts, die jeweilige Burst-Frequenzen haben, zu evaluieren; und
eine Ziel-Burst-Frequenz der Burst-Stimulation zu bestimmen, die eine größere Symptomlinderung induziert als eine Burst-Stimulation, die eine oder mehrere andere Burst-Frequenzen hat.

13. System zur Abgabe von Elektrostimulation an einen Patienten, aufweisend:
einen implantierbaren Stimulator (704), der konfiguriert ist, um Burst-Stimulation an ein neurales Target des Patienten abzugeben, wobei die Burst-Stimulation einen Pulszug gefolgt von einer pulsfreien Zeit aufweist; und
eine Steuerschaltung (820), die konfiguriert ist, um:
Patientenreaktionen auf Burst-Stimulation, die an das neurale Target gemäß einem Stimulationsparameter abgegeben wird, zu evaluieren; und
einen Hinweis auf Frequenzdrift einer intrinsischen oszillatorischen neuralen Aktivität in dem Patienten auf Basis der Patientenreaktion zu bestimmen.

14. System nach Anspruch 13, wobei die Steuerschaltung (820) konfiguriert ist, um die Patientenreaktion zu evaluieren und die Frequenzdrift periodisch oder in vorgesehenen Intervallen zu bestimmen.

15. System nach Anspruch 13, wobei zum Evaluieren der Patientenreaktion auf die Burst-Stimulation die Steuerschaltung (820) konfiguriert ist, um jeweilige Symptomlinderungsgrade als Reaktion auf jeden von mehreren Teststimulation-Bursts, die gemäß jeweiligen Werten des Burts-Stimulation-Parameters abgegeben worden sind, zu bestimmen.

## Revendications

1. Système destiné à fournir une électrostimulation à un patient, comprenant :
un stimulateur implantable (704) configuré pour fournir une stimulation par salves à une cible neurale du patient, la stimulation par salves comprenant un train d'impulsions suivi d'une période sans impulsion ; et
un circuit de commande (820) configuré pour :
recevoir une indication d'une dérive de fréquence d'une activité neurale oscillatoire intrinsèque chez le patient ;
déterminer ou ajuster un paramètre de stimulation par salves, comprenant un minutage pour initier une stimulation par salves à une phase particulière d'un cycle d'oscillation de l'activité neurale oscillatoire intrinsèque, en réponse à l'indication reçue de dérive de fréquence ; et
générer un signal de commande vers le stimulateur implantable (704) pour fournir une stimulation par salves selon le paramètre de stimulation par salves déterminé ou ajusté.

2. Système selon la revendication 1, dans lequel le paramètre de stimulation par salves comprend une fréquence de salves (826).

3. Système selon l'une quelconque des revendications 1 à 2, dans lequel le stimulateur implantable (704) comprend une stimulation cérébrale profonde (DBS) implantable configurée pour fournir une stimulation par salves à une cible cérébrale du patient.

4. Système selon l'une quelconque des revendications 1 à 3, dans lequel le circuit de commande (820) est configuré pour détecter la dérive de fréquence de l'activité neurale oscillatoire intrinsèque à l'aide d'informations physiologiques détectées chez le patient.

5. Système selon l'une quelconque des revendications 1 à 4, dans lequel, pour déterminer ou ajuster le paramètre de stimulation par salves, le circuit de commande (820) est configuré pour :
évaluer les réponses respectives du patient à chacune d'une pluralité de salves de stimulation d'essai délivrées selon des valeurs respectives du paramètre de stimulation par salves ;
déterminer une direction (1020) d'ajustement sur la base des réponses respectives du patient ; et
ajuster le paramètre de stimulation par salves selon la direction (1020) déterminée.

6. Système selon la revendication 5, dans lequel le circuit de commande (820) est configuré pour déterminer une valeur cible du paramètre de stimulation par salves sur la base des réponses respectives du patient.

7. Système selon l'une quelconque des revendications 5 à 6, comprenant au moins l'un d'un dispositif portable configuré pour détecter les réponses respectives du patient à chacune de la pluralité de salves de stimulation d'essai, ou un dispositif d'interface utilisateur configuré pour recevoir des entrées utilisateur concernant les réponses respectives du patient à chacune de la pluralité de salves de stimulation d'essai.

8. Système selon l'une quelconque des revendications 5 à 7, dans lequel, pour évaluer les réponses respectives du patient, le circuit de commande (820) est configuré pour augmenter ou diminuer progressivement les valeurs du paramètre de stimulation par salves, et pour commander le stimulateur implantable (704) afin de délivrer la pluralité de salves de stimulation d'essai selon les valeurs du paramètre de stimulation par salves augmentées ou diminuées progressivement.

9. Système selon l'une quelconque des revendications 5 à 8, dans lequel les réponses respectives du patient comprennent des niveaux respectifs de soulagement des symptômes en réponse à chacune de la pluralité de salves de stimulation d'essai, et
dans lequel le circuit de commande (820) est configuré pour déterminer une valeur cible du paramètre de stimulation par salves comme étant celle qui présente un niveau de soulagement des symptômes supérieur à une ou plusieurs autres valeurs du paramètre de stimulation par salves.

10. Système selon la revendication 9, dans lequel le circuit de commande (820) est configuré pour :
générer un modèle de prédiction de paramètre (1010) représentant une relation entre les valeurs du paramètre de stimulation par salves et les niveaux respectifs de soulagement des symptômes ; et
prédire la valeur cible du paramètre de stimulation par salves à l'aide du modèle de prédiction de paramètre (1010).

11. Système selon l'une quelconque des revendications 9 à 10, dans lequel le paramètre de stimulation par salves comprend un minutage pour initier la stimulation par salves à une phase particulière d'un cycle d'oscillation de l'activité neurale oscillatoire intrinsèque, et le circuit de commande (820) est configuré pour :
évaluer les niveaux respectifs de soulagement des symptômes en réponse à la pluralité de salves de stimulation d'essai initiées à des phases respectives du cycle d'oscillation ; et
déterminer une phase cible du cycle d'oscillation pour initier la stimulation par salves qui induit un soulagement des symptômes supérieur à celui induit par une stimulation par salves initiée à une ou plusieurs autres phases du cycle d'oscillation.

12. Système selon l'une quelconque des revendications 9 à 11, dans lequel le paramètre de stimulation par salves comprend une fréquence de salves (826), et le circuit de commande (820) est configuré pour :
évaluer les niveaux respectifs de soulagement des symptômes en réponse à la pluralité de salves de stimulation d'essai ayant des fréquences de salves respectives ; et
déterminer une fréquence de salves cible de la stimulation par salves qui induit un soulagement des symptômes supérieur à celui induit par une stimulation par salves ayant une ou plusieurs autres fréquences de salves.

13. Système destiné à fournir une électrostimulation à un patient, comprenant :
un stimulateur implantable (704) configuré pour fournir une stimulation par salves à une cible neurale du patient, la stimulation par salves comprenant un train d'impulsions suivi d'une période sans impulsion ; et
un circuit de commande (820) configuré pour :
évaluer les réponses du patient à la stimulation par salves délivrée à la cible neurale selon un paramètre de stimulation ; et
déterminer une indication d'une dérive de fréquence d'une activité neurale oscillatoire intrinsèque chez le patient sur la base de la réponse du patient.

14. Système selon la revendication 13, dans lequel le circuit de commande (820) est configuré pour évaluer la réponse du patient et déterminer la dérive de fréquence périodiquement ou à des intervalles programmés.

15. Système selon la revendication 13, dans lequel, pour évaluer la réponse du patient à la stimulation par salves, le circuit de commande (820) est configuré pour déterminer les niveaux respectifs de soulagement des symptômes en réponse à chacune d'une pluralité de salves de stimulation d'essai délivrées selon des valeurs respectives du paramètre de stimulation par salves.
